# EUROPEAN PATENT APPLICATION

(11) **EP 1 550 377 A1**
(43) Date of publication of application: **06.07.2005**
(21) Application number: 03751413.0
(22) Date of filing: 09.10.2003
(51) Int. Cl.: A23L 1/30, A23L 1/327

(54) **FOOD COMPOSITIONS FOR RECOVERY FROM FATIGUE**

(30) Priority: 11.10.2002 JP 2002298905
(71) Applicant: Ajinomoto Co., Inc., Tokyo 104-8315 (JP)
(72) Inventor: MURAKAMI, Hitoshi, Ajinomoto Co., Inc., Kawasaki-shi, Kanagawa 210-8681 (JP); YAMADA, Keiko, Ajinomoto Co., Inc., Kawasaki-shi, Kanagawa 210-8681 (JP); OHTA, Yoshizu, Ajinomoto Co., Inc., Kawasaki-shi, Kanagawa 210-8681 (JP)
(74) Representative: Strehl Schübel-Hopf & Partner
(86) International application number: PCT/JP2003/012957
(87) International publication number: WO 2004/032652

(57) **Abstract**

In the present application are disclosed a food composition for recovery from fatigue, comprising, as an effective ingredient, an extract of migratory fish such as a *Perciformes Scombrina,* or a composition of the make-up similar to the extract, according to which food composition can be provided a food composition for recovery from fatigue that enables immediate recovery from changes in the body after performing exercise or physically-demanding work, or upon waking up early in the morning, enables the body to immediately recover from a state of fatigue (decrease in spontaneous movement and liver ATP), and facilitates replenishment of the body's energy levels.

## Description

### (Technical Field)

The present invention relates to a food composition for recovery from fatigue such as an agent for recovery from fatigue, a supplement, a condiment and the like, which composition comprises an extract of migratory fish or a mixture of nutritional components of a composition similar thereto, as an effective ingredient, and which composition is effective for those who feel fatigue in daily life, are worn-out due to strenuous or long term exercise or work, or feel an unidentified complaint upon waking up.

### (Background Art)

Conventional agents for recovery from fatigue have been widely used by people in modern society (here, the term "people in modern society" refers to all kinds of people including healthy subjects and those suffering from illness) in the form of quasi-drugs or nourishing drinks containing vitamins or caffeine, or natural nourishing food products such as Asian ginseng, royal jelly, propolis, or the like. However, the functional mechanisms of these foods have not been completely clarified, and it is hard to say that the effects thereof have been sufficiently realized for human. Further, these existing foods are generally provided in a pharmaceutical form, and it is difficult to utilize them by mixing them in existing foods because of their functional properties and physicochemical properties. If the purpose is to ingest such type of foods on a routine basis in every day dietary life in order to recover from fatigue or prevent it, it is believed that the food should, if possible, preferably be not only in the form of a supplement but also be capable of being mixed in general foods to provide a delicious taste, and a food that has been subjected to widespread ingestion experience (the term "widespread ingestion experience" referring to a food having been widely eaten since ancient times) is more preferable. From these viewpoints, since components of the above-mentioned existing foods, drugs and supplements have limited application to foods, there has been a desire for the development of a food that has versatility, plentiful ingestion experience, and that is more effective as a food.

When a living body performs intensive exercise or work, the body loses a lot of energy. It has been known since a long time ago that carbohydrates and fats are preferentially used as energy sources for recovery of the lost energy. The rate-determining step for energy production is ATP-producing enzymes in the liver and muscles, and sufficient production of ATP therein is extremely important for smooth energy metabolism and avoidance of a fatigued state.

The results of tests performed by the present inventors (Test Examples described later being referred to) showed that, when a laboratory animal is loaded with an exercise for a long time, concentration of the ATP in the liver reduces immediately and, along with this, spontaneous activity of the laboratory animal decreases significantly, although the ATP in skeletal muscles do not show changes. Since it has been found in the present invention that extracts from a bonito, which is a kind of migratory fish, was extremely effective in these cases for promptly recovering the liver ATP concentration and normalizing the spontaneous activity of an animal, the extracts are believed to be effective for recovering from fatigue after exercise or work, and for improvement of an unidentified complaint when getting up early in the morning.

On the other hand, although nourishing drinks that have been widely used conventionally for recovery from fatigue contain, as main ingredients, various kinds of vitamins, caffeine, taurine and the like no ingredients other than caffeine exhibit a distinct effect for recovery from fatigue. Further, as for Asian ginseng, royal jelly and propolis protein, even when an effect is recognized, the active site (here, the term "active site" referring to a biochemical target of a potent ingredient, e.g., a specific enzyme) is not clear. In addition, all of these products have a large problem with regard to sensory feeling, and it is believed that use of them in a form other than that of a pharmaceutical is rather difficult and their frequent use in everyday dietary life is difficult.

On the other hand, it is known that anserine and carnosine contained in large quantities in seafood and animal meat activate ATPase, and JP-A-2002-173422 discloses that an enhancement of exercise capacity and anti-fatigue effect are exhibited by administrating at least one kind selected from imidazole peptides, especially anserine, carnosine and balenine obtained by purifying specifically lower molecular fractions of extracts of seafood, chicken meat, animal meat or the like, by allowing them to run through an ultrafilter membrane. However, there is disclosed no discussion of the amount of ATP that is important upon recovery from fatigue after an exercise load, and therefore the relationship between administration and recovery from fatigue is not clear.

### (Disclosure of the Invention)

In the background of the background art described in the previous section, it is the object of the present invention to provide a food composition for recovery from fatigue that enables immediate recovery from changes in the body after performing exercise or physically-demanding work, or upon waking up early in the morning, enables the body to immediately recover from a state of fatigue (decrease in spontaneous movement and liver ATP), and facilitates replenishment of the body's energy levels.

The present inventors have conducted concentrated studies to achieve the aforementioned object, and found as the results that an extract from migratory fish such as bonito, tuna and mackerel and a mixture of nutritional components having a composition similar thereto restores immediately a liver ATP amount and also recovers from a fatigued state. They have completed the present invention on the basis of these findings.

Accordingly, the present invention relates to a food composition for recovery from fatigue, comprising, as an effective ingredient, an extract of migratory fish such as bonito, tuna, mackerel or the like, and also to a food composition for recovery from fatigue, comprising, as an effective ingredient, a mixture of nutritional components, said mixture containing (a) a total of 294 mg or more of nitrogen-containing compounds such as peptides, proteins, amino acids and the like derived from a bonito extract, or (a') 4 mg or more of each of amino acids (76 mg or more in terms of a total amino acid amount) including taurine, asparatic acid, threonine, serine, glutamic acid, proline, glycine, alanine, valine, methionine, isoleucine, leucine, tyrosine, phenylalanine, lysine, histidine, anserine, carnosine and arginine; (b) a total of 55 mg or more of the organic acids consisting of lactic acid, citric acid, malic acid and succinic acid; (c) 1 mg or more of nucleic acid-related compounds; and (d) a total of 16 mg or more of mineral components such as NaCl and KCl, at a ratio by weight of, when the amino acid amount is defined as 1, from 0.5 to 2 of the organic acid, from 0.001 to 0.5 of the nucleic acid-related compounds and from 0.02 to 2 of the mineral component.

Hereinafter, the present invention will be described in greater detail.

The term "migratory fish" as used in connection with the present invention refers to fish that carry out a large migration on a regular basis for the purpose of egg-laying or according to the season, including bonito, tuna, swordfish, mackerel, sardine, herring, yellow-tail and the like. Use of fish belonging to *Perciformes Scombrina* such as bonito, mackerel, bullet mackerel, butter fish, southern bluefin tuna, tuna, bluefin tuna, albacore, bigeye tuna, swordfish and the like, is especially preferable in view of the positive effect thereof. Here, this classification is based on "Genshoku Gyorui Kensaku Zukan (Originally Colored Picture Book for Searching Fish) (revised 13^{th} edition)" (published by Hokuryukan in 1989).

A method for producing the extract of a migratory fish for use according to the present invention is not particularly limited, and the extract may be obtained by an ordinary method, or an extract produced by a method different from an ordinary method may be used as long as it has a similar composition.

More specifically, the extract of a migratory fish usable in the present invention includes an extract obtained, for example, by concentrating a cooked broth and/or a steam-cooked broth of fish meat such as bonito, tuna, mackerel or the like to around Brix 20 to 40 according to an ordinary method, filtrating, followed by hydrolyzing enzymatically the filtrate with a protease at 20 to 60 °C for a period within a range of 1 minute to 24 hours, and concentrating the resulting product to around Brix 40 to 60, adding sugars (such as glucose, fructose and the like) within a range of 0.1 to 10 % and, subsequently, subjecting the resulting product to heat-browning processing at 60 to 150 °C for a period within a range of 1 minute to 24 hours. Further, there may be mentioned as one example, an extract that is prepared by mincing entrails, fish meat and heads of the aforementioned migratory fish, followed by *koji* mold fermentation , pasteurization and filtration (JP-A-2002-191321). As a further example, there may be mentioned an extract that is obtained by heat-concentrating a cooked broth and/or steam-cooked broth of fish meat of the aforementioned migratory fish to around Brix 20 to 40 according to an ordinary method, followed by filtrating the same.

In addition, the aforementioned extracts may be mixed with other energy-generating sources such as sugars or the like.

This type of extract may be one from which the salt content has been removed by a reverse osmosis filtration method or the like.

In place of an extract of the migratory fish such as bonito, tuna, mackerel, or the like, a composition having a composition similar to that of the extract can also be employed. Examples of the composition include a composition (Composition X) containing, as an effective ingredient, a mixture of nutritional components comprising (a) a total of 294 mg or more of nitrogen-containing compounds such as peptides, proteins, amino acids, and the like, derived from an extract of bonito, tuna or mackerel, or the like, (b) a total of 55 mg or more of organic acids consisting of lactic acid, citric acid, malic acid and succinic acid, (c) 1 mg or more of nucleic acid-related compounds and (d) 16 mg or more in total of mineral components such as NaCl and KCl, by a weight ratio of, when the amount of the amino acids is defined as 1, from 0.5 to 2 of the organic acid, from 0.001 to 0.5 of the nucleic acid-related compounds and from 0.02 to 2 of the mineral components; and a composition (Composition Y) containing, as an effective ingredient, a mixture of nutritional components comprising (a') 4 mg or more each of the amino acids consisting of taurine, asparatic acid, threonine, serine, glutamic acid, proline, glycine, alanine, valine, methionine, isoleucine, leucine, tyrosine, phenylalanine, lysine, histidine, anserine, carnosine and arginine (76 mg or more of the amino acids in the total amount), (b) a total of 55 mg or more of the organic acids consisting of lactic acid, citric acid, malic acid and succinic acid, (c) 1 mg or more of nucleic acid-related compounds, and (d) 16 mg or more in total of mineral components such as NaCl and KCl, by a weight ratio of, when defining the amount of the amino acids as 1, from 0.5 to 2 of the organic acid, from 0.001 to 0.5 of the nucleic acid-related compounds and from 0.02 to 2 of the mineral components. In this connection, the term "nucleic acid-related compounds" in such composition refers to nucleic acids, nucleotides, nucleosides and nucleic acid bases.

Comparing Composition X with Composition Y, Composition X is superior to Composition Y in terms of the recovery from fatigue effect. Composition X is presumed to contain an unknown component (such as a Maillard reaction product or the like) that is thought to be contained in a bonito extract, which may cause superiority with respect to the recovery from fatigue effect.

In addition to an extract of migratory fish or a mixture of nutritional components having a composition similar to that of the extract, the food composition for recovery from fatigue according to the present invention may be compounded with suitable additives in such a range that the effect of the present invention is not jeopardized.

Examples of such additives include other nutritional components that promote the fatigue recovering effect of the migratory fish extract such as carbohydrates (glucose, sucrose, starch etc.), lipids (vegetable oil, fish oil, animal fat etc.), proteins (soybean protein, milk protein etc.), minerals (inorganic salts such as potassium salt, sodium salt and calcium salt etc.), vitamins (thiamine, niacin, vitamin C, carotene etc.); anti-fatigue components such as taurine, caffeine etc.; taste-improving components such as sucrose, aspartame, acesulfame K, sodium glutamate, sodium chloride etc. suitable for imparting functions as a food (taste, eating feeling, safety etc.); excipients such as carbohydrates, inorganic salts etc. for granulation, powdering or solidification for the similar purpose of rendering the composition easy to eat; bacteriostatic components such as ethyl alcohol, acetic acid, sodium acetate, glycine etc. for a similar purpose; and pigments including pigments derived from natural products such as annatto pigment, safflower pigment, paprika pigment, *beni-koji (*red *koji* mold*)* pigment, grape pigment etc. and various synthetic pigments for a similar purpose. An additive is not limited to these, and other additives that are conventionally used in this field can also be compounded.

The nutritional composition according to the present invention manufactured in the above-described manner can be distributed without further modification, that is, in the form of a liquid mixture, a powder mixture or the like, as the case may be. In addition, it can also be distributed in the form of a recovery from fatigue agent, a supplement, a condiment or the like.

Finally, a dose (intake) of the nutritional composition according to the present invention will be described. The results of the administration tests of a bonito extract to mice showed that the recovery from fatigue action was observed when the dosage exceeded 630 mg/kg in terms of the dry matter. When the dosage for an animal is converted to a dosage for humans, the dosage is 500 mg or more per meal for adult humans in terms of the dry matter, assuming that the humans eat three meals per day. When the dosage is less than this amount, no effect can be expected.

### (Brief Description of the Drawings)

Fig. 1 shows the change in spontaneous motor activity (Test Example 1).
Fig. 2 shows the ATP/AMP ratio in the liver (Test Example 1).
Fig. 3 shows the change in spontaneous motor activity (Test Example 2).
Fig. 4 shows the ATP/AMP ratio in the liver (Test Example 3).
Fig. 5 shows the ATP/AMP ratio in the liver (Test Example 4).
Fig. 6 shows the change in spontaneous motor activity (Test Example 5).
Fig.7 shows the ATP/AMP ratio in the liver (Test Example 5).
Fig. 8 shows the change in spontaneous motor activity (Test Example 6).
Fig. 9 shows the change in spontaneous motor activity (Test Example 6).
Fig. 10 shows the change in spontaneous motor activity (Test Example 6).
Fig. 11 shows the change in spontaneous motor activity (Test Example 7).
Fig.12 shows the spontaneous motor activity after continuous ingestion for a week (Test Example 8).

### (Best Mode for Carrying Out the Invention)

Hereinafter, the invention will be described in further detail on the basis of Examples and Test Examples.

### Example 1: Preparation of an bonito extract

Three kinds of bonito extracts were prepared. That is, first, Bonito Extract A was prepared by concentrating a cooked broth of bonito meat to around Brix 30 according to an ordinary method followed by filtering. The filtrate was subjected to an enzymatic hydrolysis with a protease at 50 °C for 3 hours, followed by heating the resulting mass at 90 °C for 10 minutes to deactivate the enzyme and concentrating to around Brix 60. The concentrate was added with 2 % of sugar (glucose) and subjected to a heat-browning treatment. Next, Bonito Extract B was prepared by the steps of mincing bowels, meat and heads of bonitos, subjecting the minced product to *koji* mold-fermentation, followed by pasteurizing and filtering. Finally, Bonito Extract C was prepared by heat-concentrating a cooked broth of bonito meat to around Brix 30 according to an ordinary method, followed by filtering.

### Test Example 1: Recovery of spontaneous activity and ATP/AMP ratio in liver

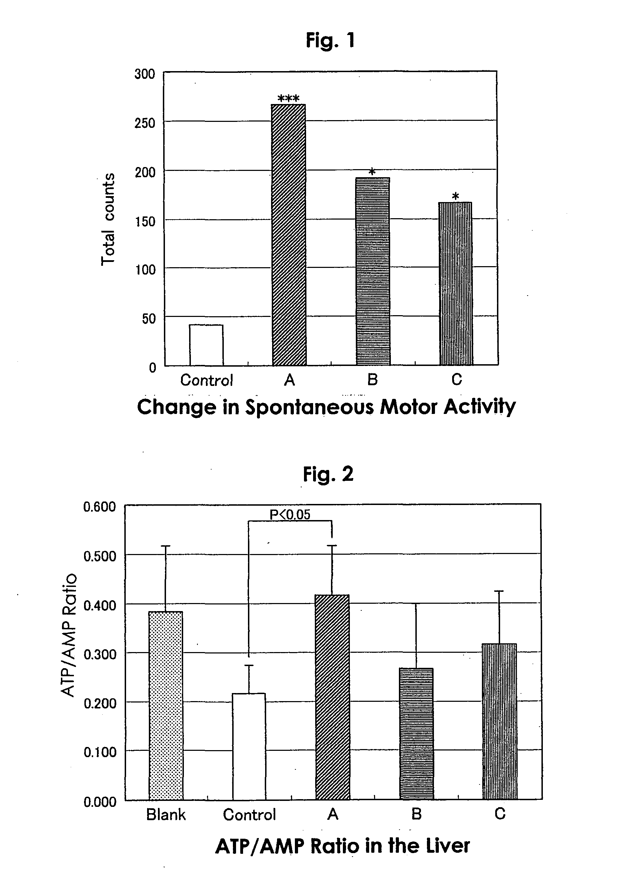

Three groups (A, B and C groups) of 5-week-old CDF1 male mice, each group consisting of 8 mice (n=8 for each group), were put in a treadmill and subjected to forced walking exercise for 3 hours. Then, the mice of the respective groups were orally administrated, using a gastric sonde, with (1) Bonito Extract A, (2) Bonito Extract B, and (3) Bonito Extract C in an amount of 1 g/kg in terms of free amino acid amount in the bonito extracts. Subsequently, their spontaneous activity for 60 minutes was measured using a spontaneous activity measuring apparatus provided with an infrared sensor, and comparison with the control group (Control group which was administrated with only deionized distilled water in the same manner as the samples) was carried out. The respective samples and the distilled water were administered in amounts of 0.5 mL. The result is represented in Fig. 1.

In the Figure, *, ** and *** represent significant differences from the control group, * being at p<0.05, ** being at p<0.01, and *** being at p<0.001. "Total counts" refers to the number of times of exercising that were measured with the infrared sensor for 60 minutes.

Further, three groups (A, B and C groups) of 5-week-old CDF1 male mice, each group consisting of 6 mice, were put in a treadmill and subjected to forced walking exercise for 5 hours. The three groups were administered, 30 minutes before the end of the exercise, with the aforementioned Bonito Extracts A to C, respectively, and after the end of the exercise the mice were sacrificed to measure the ATP/AMP ratio in the liver. For comparison, the ATP/AMP ratio was measured for a group that was subjected to neither exercise load nor administration (Blank group), and for another group that was subjected to the exercise load but administered with distilled water (Control group). Here, the liver ATP and AMP were measured according to the method of Carter et al. described in Journal of Chromatography, vol. 527, P. 31-39 (1990). The result is represented in Fig. 2.

As can be seen from Fig. 1, while recovery of spontaneous activity was observed for administration of any one of the bonito extracts, the effect was, among them, recognized especially for the Bonito Extract A that had been subjected to the enzyme treatment. In addition, as shown in Fig. 2, Bonito Extract A restored significantly the ATP/AMP ratio in the liver, too.

### Test Example 2: Recovery of spontaneous activity

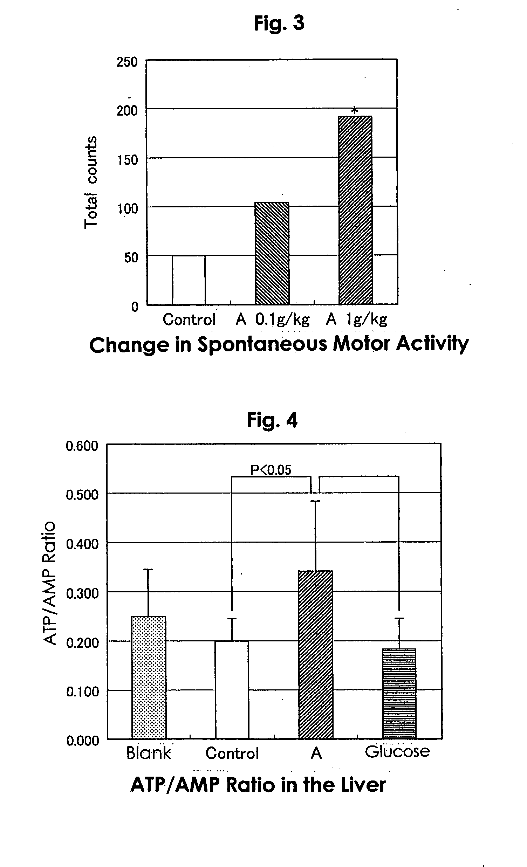

Three groups of 5-week-old CDF1 male mice, each group consisting of 8 mice (n=8 for each group), were put in a treadmill and subjected to forced walking exercise for 3 hours. Then, the mice of the respective groups were orally administrated, using a gastric sonde, with (1) the Bonito Extract A in an amount of 0.1 g/kg in terms of a free amino acid amount (A 0.1 g/kg group), and (2) the Bonito Extract A in an amount of 1 g/kg in terms of a free amino acid amount (A 1 g/kg group). Subsequently, spontaneous activity for 60 minutes was measured using a spontaneous activity measuring apparatus provided with an infrared sensor, and comparison with a control group (Control group which was administrated with only deionized distilled water in the same manner as the samples) was carried out. The respective samples and the distilled water were administered in amounts of 0.5 mL. The result is represented in Fig. 3. In the Figure, * represents a significant difference from Control group, * being at p<0.05.

As can be seen from Fig. 3, significant recovery of spontaneous activity was recognized in the group administrated with the Bonito Extract A in an amount of 1 g/kg in terms of a free amino acid amount, and a tendency to recover spontaneous activity was also recognized for the group administrated with 0.1 g/kg, which is 1/10 the amount of the 1 g/kg group.

### Test Example 3: Recovery of the ATP/AMP ratio in the liver

Two groups of 5-week-old CDF-1 male mice, each group consisting of 6 mice, were put in a treadmill and subjected to forced walking exercise for 5 hours. The mice of the respective groups were, 30 minutes before the end of the exercise, administrated with (1) the Bonito Extract A in an amount of 1 g/kg in terms of a free amino acid amount (A group), and (2) D-glucose in an amount of 1 g/kg (Glucose group). After the end of the exercise, they were sacrificed to measure the ATP/AMP ratio in the liver. For comparison, the ATP/AMP ratio was measured for a group that had been subjected to neither exercise load nor administration (Blank group), and for a group that had been subjected to the exercise load but administered with distilled water (Control group). The conditions of the administration method and administration amount were the same as those described in Test Example 1.

The result is represented in Fig. 4. As can be seen from Figure 4, the Bonito Extract A can restore the ATP/AMP ratio in the liver more significantly than glucose.

### Test Example 4: ATP/AMP ratio in the liver

Three groups of 5-week-old CDF-1 male mice, each group consisting of 6 mice, were put in a treadmill and subjected to forced walking exercise for 5 hours. The mice of the respective groups were, 30 minutes before the end of the exercise, administered with (1) the aforementioned Bonito Extract A in an amount of 1 g/kg in terms of a free amino acid amount (A group), (B) a bonito essence "Marine Active" (made by Yaizu Suisankagaku Industry Co., Ltd.) in an amount of 1 g/kg in terms of a free amino acid amount contained therein (Marine Active group), and (3) anserine in an amount of 200 mg/kg (Anserine group), which is an amount that is recognized to have an anti-fatigue effect in the aforementioned JP-A-2002-173442. After the end of the exercise, they were sacrificed to measure the ATP/AMP ratio in the liver. For comparison, the ATP/AMP ratio was measured for mice in a group that had been subjected to neither exercise load nor administration (Blank group), and in a group that had been subjected to the exercise load but administered with distilled water (Control group). The conditions of the administration method and administration amount were the same as those described in Test Example 1.

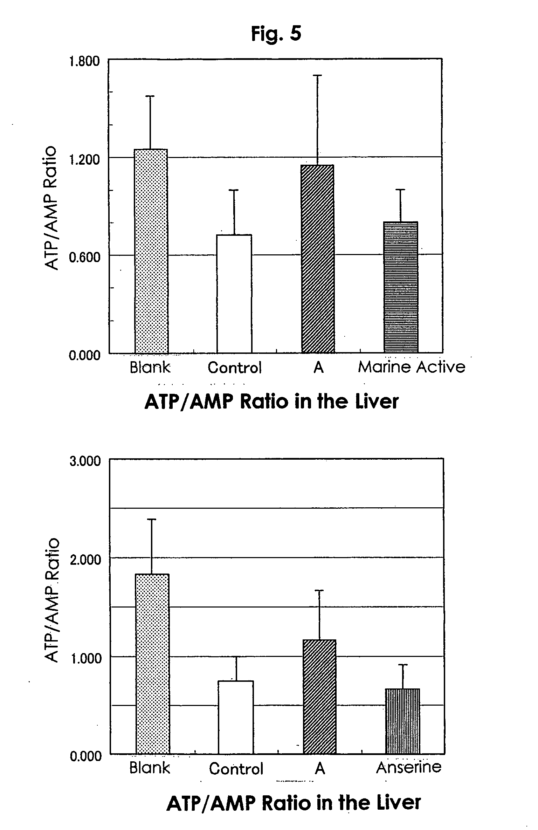

The result is represented in Fig. 5. As can be seen from Fig. 5, it was revealed that the group administered with the Bonito Extract A exhibited a higher level of the ATP/AMP ratio in the liver compared with the group administered with Marine Active, which is a product manufactured according to a conventional technique, and the group administrated with anserine, which is said to have an anti-fatigue effect.

### Test Example 5: Spontaneous activity and the ATP/AMP ratio in the liver

Two groups of 5-week-old CDF1 male mice, each group consisting of 8 mice (n=8 for each group), were put in a treadmill and subjected to forced walking exercise for 3 hours. Then, the mice of the respective groups were orally administrated with (1) the Bonito Extract A in an amount of 1 g/kg in terms of a free amino acid amount (A group), and (2) an amino acid mixture of the free amino acid composition in the Bonito extract A (a mixture of taurine, asparatic acid, threonine, serine, glutamic acid, proline, glycine, alanine, valine, methionine, isoleucine, leucine, tyrosine, phenylalanine, lysine, histidine, anserine, carnosine and arginine) in an amount of 1 g/kg (AAMix group). Subsequently, spontaneous activity for 60 minutes was measured using a spontaneous activity measuring apparatus provided with an infrared sensor, and comparison with a control group (Control group which had been administrated with only deionized distilled water) was carried out. The conditions of the administration method and administration amount were the same as those described in Test Example 1.

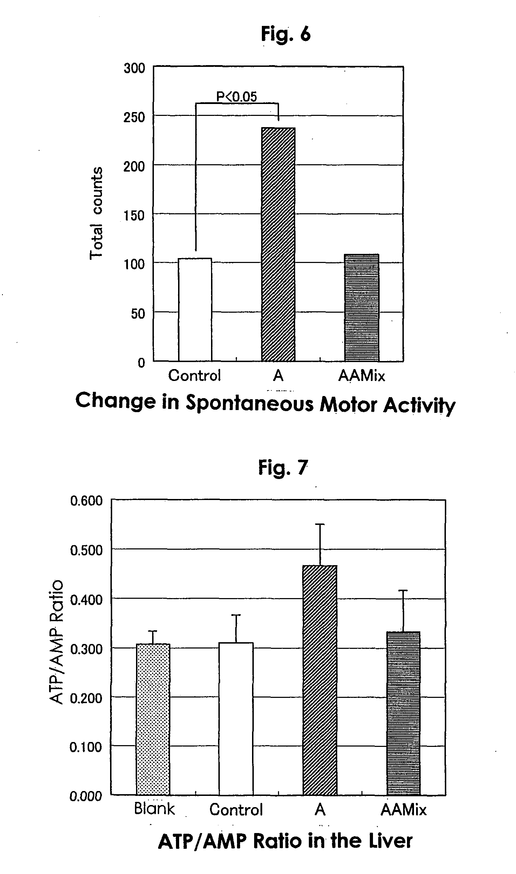

The result is represented in Fig. 6.

Further, two groups of 5-week-old CDF-1 male mice, each group consisting of 6 mice, were put in a treadmill and subjected to forced walking exercise for 5 hours. They were administered, 30 minutes before the end of the exercise, with the aforementioned (1) and (2), respectively. After the end of the exercise, they were sacrificed to measure the ATP/AMP ratio in the liver. For comparison, the ATP/AMP ratio was also measured for a group that had been subjected to neither exercise load nor administration (Blank group), and for a group that had been subjected to the exercise load but administered with distilled water (Control group). The result is represented in Fig. 7.

As can be seen from Figs. 6 and 7, although recovery of spontaneous activity and the ATP/AMP ratio in the liver were observed for the group administrated with the Bonito Extract A, no change was observed for the group administrated with the mixture of the free amino acid composition in the Bonito Extract A. It is thus understood that a certain substance included in the bonito extract other than the constitutional amino acid mixture is necessary.

### Test Example 6: Change in spontaneous activity

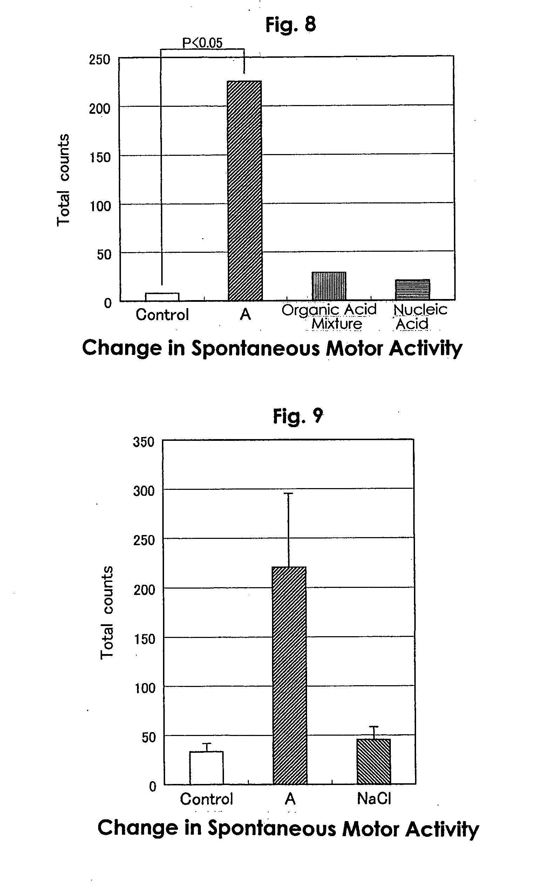

Five groups of 5-week-old CDF1 male mice, each group consisting of 8 mice (n=8 for each group), were put in a treadmill and subjected to forced walking exercise for 3 hours. Then, the mice of the respective groups were orally administrated with a known component or components of the Bonito Extract A, that is, (1) a 550:4:3:5 mixture of lactic acid, citric acid, malic acid and succinic acid (Organic acid mixture group), (2) inosine 1-phosphate (Nucleic acid group), (3) NaCl (NaCl group) and (4) a mixture of (1) to (3) added with the amino acid mixture used in Test Example 5 (Amino acid mixture/organic acid mixture/NaCl group), and (5) the Bonito Extract A in an amount of 1 g/kg in terms of a free amino acid amount (A group). Subsequently, spontaneous activity for 60 minutes was measured using a spontaneous activity measuring apparatus provided with an infrared sensor, and comparison with a control group (Control group which had been administrated with only deionized distilled water) was carried out. The conditions of the administration method and administration amount were the same as those described in Test Example 1. The result is represented in Figs. 8, 9 and 10.

Figs. 8, 9 and 10 show that, although recovery of spontaneous activity was observed for the group administrated with the Bonito Extract A, recovery of spontaneous activity was not observed for the groups administrated with the free amino acid mixture with the composition in the Bonito Extract A, the organic acid mixture, the nucleic acid or NaCl. However, for the group administrated with the mixture of all of these, recovery of spontaneous activity was observed in comparison with the Control group, although it was not as significant as that of the group administrated with the Bonito Extract A.

### Example 2: Preparation of a tuna extract and a bonito extract

A tuna hot water extract was prepared according to the following method. That is, first, 1 kg of minced meat of tuna (bluefin tuna) was added with 2 kg of hot water to carry out extraction at 95 to 97 °C for 1 hour. The cooked broth obtained by filtration was freeze-dried by an ordinary method to give 42 g of hot water-extracted tuna essence powder.

A bonito hot water extract was also prepared according to a similar method. That is, 1 kg of minced meat of bonito was added with 2 kg of hot water to carry out extraction at 95 to 97 °C for 1 hour. The cooked broth obtained by filtration was freeze-dried by an ordinary method to give 40 g of hot water-extracted bonito essence powder.

### Test Example 7: Measurement of spontaneous activity

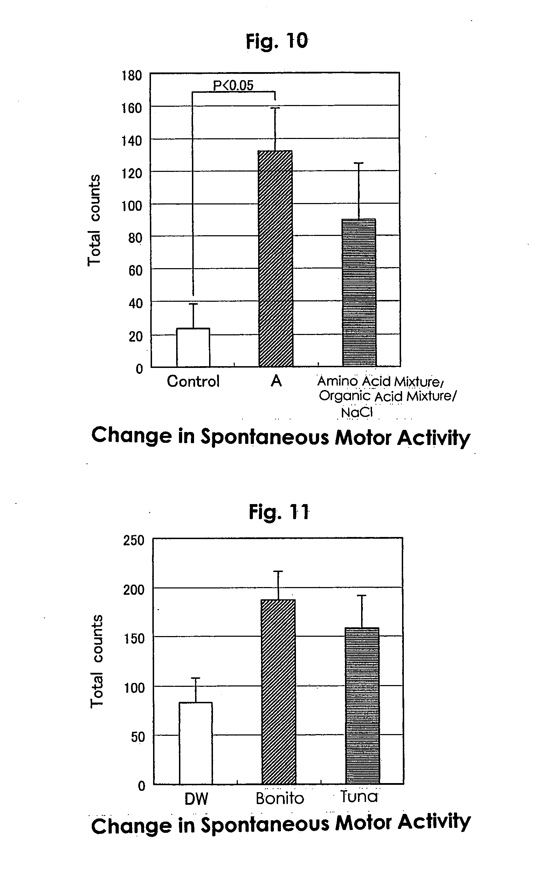

Three groups (A, B and C group) of 5-week-old CDF1 male mice, each group consisting of 8 mice (n=8 for each group), were put in a treadmill and subjected to forced walking exercise for 3 hours. Then, the mice of the respective groups were orally administrated with (1) distilled water (DW), (2) the hot water-extracted bonito essence obtained in Example 2 (in an amount of 1 g/kg in terms of a free amino acid amount), and (3) the hot water-extracted tuna essence obtained in Example 2 (in an amount of 1 g/kg in terms of a free amino acid amount). Subsequently, spontaneous activity for 60 minutes was measured using a spontaneous activity measuring apparatus provided with an infrared sensor, and comparison with a control group (Control group which had been administrated with only deionized distilled water) was carried out. The conditions of the administration method and administration amount were the same as those described in Test Example 1. The result is represented in Fig. 11.

As shown in Fig. 11, in comparison with the group administrated with distilled water (DW group), recovery of spontaneous activity was observed for both the group administrated with the hot water-extracted tuna essence and the group administered with the hot water-extracted bonito essence.

### Test Example 8: Preparation of a bonito extract and measurement of spontaneous activity

A cooked broth of bonito meat was subjected to vacuum concentration to Brix 30 according to an ordinary method, followed by filtration. The filtrate was then subjected to electrodialysis ("Acilyzer G3", made by Asahi Kasei Corporation) and freeze-drying whereby a desalted bonito extract was obtained.

An animal test was carried out according to the following method. Two groups of 5-week-old CDF1 male mice, each group consisting of 10 mice, were administered, respectively, with (1) Control feed (a purified feed having the composition of "AIN-93G"; Journal of Nutrition 123, 1939-1951, 1993) and (2) a feed added with 5 % of the bonito extract (a feed prepared by reducing the compounded amount of corn starch in the aforementioned purified feed by 5 % and replacing this amount with the bonito extract) to rear them for a week. The mice were allowed to incept the feed freely and given a load of forced walking of 3 hours per day for 5 days per week during the raising period. One week after the start of rearing, spontaneous activity was measured after the forced walking by the same method as that described above (Test Example 1) using an infrared sensor.

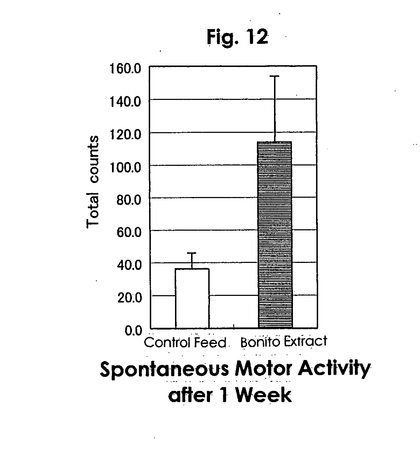

The obtained result is represented in Fig. 12. As shown in the figure, for the mice fed with the feed added with the bonito extract, a tendency to exhibit a higher level of spontaneous activity was observed compared with the mice in the group fed with the Control feed. In this connection, with respect to the amount of ingested feed, no significant difference was recognized between the groups. Based on the above result, it was confirmed that continuous ingestion of the bonito extract also exhibits the recovery from fatigue effect.

### (Industrial Applicability)

According to the present invention can be provided a food composition for recovery from fatigue that enables immediate recovery from changes in the body after performing exercise or physically-demanding work, or upon waking up early in the morning, enables the body to immediately recover from a state of fatigue (decrease in spontaneous activity and liver ATP), and facilitates replenishment of the body's energy levels.

## Claims

1. A food composition for recovery from fatigue, comprising an extract of migratory fish as an effective ingredient.

2. The food composition for recovery from fatigue according to claim 1, comprising, in addition to the extract of migratory fish, at least one additive selected from the group consisting of other nutritional components, anti-fatigue components, taste-improving components, diluting components, bacteriostatic components and coloring matters.

3. The food composition for recovery from fatigue according to claim 1 or 2, wherein the migratory fish is fish belonging to *Perciformes Scombrina.*

4. The food composition for recovery from fatigue according to claim 1 or 2, wherein the migratory fish is bonito.

5. The food composition for recovery from fatigue according to claim 1 or 2, wherein the migratory fish is tuna.

6. The food composition for recovery from fatigue according to claim 1 or 2, wherein the extract of migratory fish is an extract that is extracted from a cooked broth of bonito meat or/and tuna meat.

7. The food composition for recovery from fatigue according to claim 1 or 2, wherein the extract of migratory fish is an extract that is obtained by enzymatic hydrolysis of a cooked broth of bonito meat or/and tuna meat.

8. The food composition for recovery from fatigue according to claim 1 or 2, wherein the extract of migratory fish is an extract that is obtained by heat-concentrating a cooked broth or/and a steam-cooked broth of bonito meat and/or tuna meat.

9. The food composition for recovery from fatigue according to claim 1 or 2, wherein the extract of migratory fish is an extract that is obtained from a brew produced by brewing by-products from fish processing of bonito and/or tuna, including heads, entrails and/or abdominal skins, with the use of *koji* mold after mincing the by-products.

10. A food composition for recovery from fatigue, comprising, as an effective ingredient, a mixture of nutritional components, said mixture comprising (a) a total of 294 mg or more of nitrogen-containing compounds such as peptides, proteins, amino acids, and the like derived from a bonito extract or/and a tuna extract, or (a') 4 mg or more of each of the amino acids consisting of taurine, asparatic acid, threonine, serine, glutamic acid, proline, glycine, alanine, valine, methionine, isoleucine, leucine, tyrosine, phenylalanine, lysine, histidine, anserine, carnosine and arginine (76 mg or more in terms of a total amino acid amount) ; (b) a total of 55 mg or more of the organic acids consisting of lactic acid, citric acid, malic acid and succinic acid; (c) 1 mg or more of nucleic acid-related compounds; and (d) a total of 16 mg or more of mineral components such as NaCl and KCl, at a ratio by weight of, when the amino acid amount is defined as 1, from 0.5 to 2 of the organic acid, from 0.001 to 0.5 of the nucleic acid-related compound and from 0.02 to 2 of the mineral components.
